# EUROPEAN PATENT APPLICATION

(11) **EP 3 683 783 A1**
(43) Date of publication of application: **22.07.2020**
(21) Application number: 18854341.7
(22) Date of filing: 19.07.2018
(51) Int. Cl.: G09B 23/28

(54) **FLUID DRIVING DEVICE**

(30) Priority: 11.09.2017 CN 201710812958
(71) Applicant: Suzhou Medical Implant Mechanics Co., Ltd, Jiangsu 215123 (CN)
(72) Inventor: ZUO, Hui, Suzhou Jiangsu 215123 (CN); ZHOU, Zhenbo, Suzhou Jiangsu 215123 (CN)
(74) Representative: Cabinet Laurent & Charras
(86) International application number: PCT/CN2018/096254
(87) International publication number: WO 2019/047623

(57) **Abstract**

A fluid driving device includes: a pipe flow system configured to provide a fluid flow channel; a power system configured to provide power for the fluid to flow into and out of the pipe flow system; and a control system, configured to control the operation of the fluid driving device.

## Description

### TECHNICAL FIELD

The present disclosure relates to the field of mechanical electronic devices, for example, to a fluid driving device.

### BACKGROUND

In the related art, fluid driving devices that can generate a periodic pulsating fluid have emerged to simulate an artificial heart or the like. They are used by clinicians or medical instruments research and development enterprises to simulate the clinical use environment of a device and test a medical instrument, or they may also be used by relevant units and schools for purposes of teaching demonstrations. However, most of the fluid driving devices for simulating the heart in the related art has a simple function and cannot accurately simulate the physiological conditions of the human heart (for example, it is difficult to simulate the contraction and relaxation of the heart, the heart rate, and the cardiac output, etc., or to simulate the blood temperature and pressure), or they may have a large volume and so are not portable.

### SUMMARY

The present disclosure provides a fluid driving device that has a compact structure and is able to quickly provide a pulsating fluid as needed.

There is provided a fluid driving device that includes:
a pipe flow system, configured to provide a fluid channel;
a power system, configured to provide power for a fluid to flow into and out of the pipe flow system; and
a control system, configured to control operation of the fluid driving device.

In an embodiment, the fluid driving device further includes a base. The pipe flow system is formed inside the base. The power system is supported on the base. The control system is supported on the base. And the power system is disposed between the control system and the base.

In an embodiment, the pipe flow system includes an inner chamber defined by an interior space of the base, an inflow valve operative to control the fluid to flow into the inner chamber, and an outflow valve operative to control the fluid to flow out of the inner chamber.

In an embodiment, the inflow valve and the outflow valve are both check valves.

In an embodiment, the check valve includes a valve body, a ball cage arranged at the valve body along a flowing direction of the fluid, and a movable ball arranged inside the ball cage. The valve body includes a fluid channel, and the ball is configured to block or open the fluid channel by moving in the ball cage.

In an embodiment, the valve body is formed from a soft material, the soft material including silicone or rubber.

In an embodiment, the fluid driving device further includes an energy accumulator, which is communicated with the interior chamber of the pipe flow system and is configured to buffer the flow of the fluid and to adjust a pressure difference between a first pressure and a second pressure of the fluid, where the first pressure is a fluid pressure after the energy accumulator stores energy, and the second pressure is a fluid pressure after the energy accumulator releases energy.

In an embodiment, the power system includes:
a housing, communicated with an upper portion of the inner chamber;
a piston, movable along an up and down direction in the housing; and
a drive mechanism, disposed above the housing and configured to drive the piston to move in the up and down direction.

In an embodiment, the drive mechanism includes a linear motor, a cylinder, a solenoid valve, or a reciprocating mechanical structure.

In an embodiment, the power system includes:
a housing, communicated with an upper portion of the inner chamber; and
a deformable elastomer, connected to the housing and configured to deform in the housing so as to provide a power.

In an embodiment, the fluid driving device further includes a pressure sensor configured to detect a pressure of the fluid in the pipe flow system.

In an embodiment, the control system is configured to control the pressure of the fluid in the pipe flow system based on the pressure of the fluid detected by the pressure sensor.

In an embodiment, the fluid driving device further includes a flow sensor configured to detect a flow of the fluid in the pipe flow system.

In an embodiment, the control system is configured to control the flow of the fluid flowing into the pipe flow system based on the flow of the fluid detected by the flow sensor.

In an embodiment, the fluid driving device further includes a heating assembly connected to the pipe flow system and configured to heat the fluid in the pipe flow system.

In an embodiment, the fluid driving device further includes a temperature sensor configured to detect a temperature of the fluid in the pipe flow system.

In an embodiment, the control system is further configured to control the heating assembly depending on a detection result of the temperature sensor.

In an embodiment, the inflow valve is connected to an inflow pipe, and the outflow valve is connected to an outflow pipe, where the inflow pipe is provided with a flow control valve configured to control the rate of flow and pressure of the fluid flowing into the fluid driving device.

In an embodiment, the fluid driving device further includes a touch display screen or a portable computer electrically connected to the control system and configured to input a control command and to display the pressure and the temperature detected by the pressure sensor and the temperature sensor.

In an embodiment, the control system is further configured to control the power system and the heating assembly to stop operating when detecting no fluids in the pipe flow system.

The fluid driving device of the present disclosure has an overall simple and compact structure.

Thus, the overall instrument volume and weight, and making it convenient to carry around.

### BRIEF DESCRIPTION OF DRAWINGS

FIG. 1 is a schematic diagram of a fluid driving device according to an embodiment of the present disclosure.
FIG. 2 is a schematic diagram of a pipe flow system of FIG. 1.
FIG. 3 is a schematic diagram of an inflow valve of FIG. 2.
FIG. 4 is a schematic diagram of an outflow valve of FIG. 2.
FIG. 5 is a schematic diagram of a power system of FIG. 1.
FIG. 6 is a schematic diagram of an energy accumulator of FIG. 1.
FIG. 7 is a schematic diagram of the fluid driving device according to another embodiment of the present disclosure.

### DETAILED DESCRIPTION

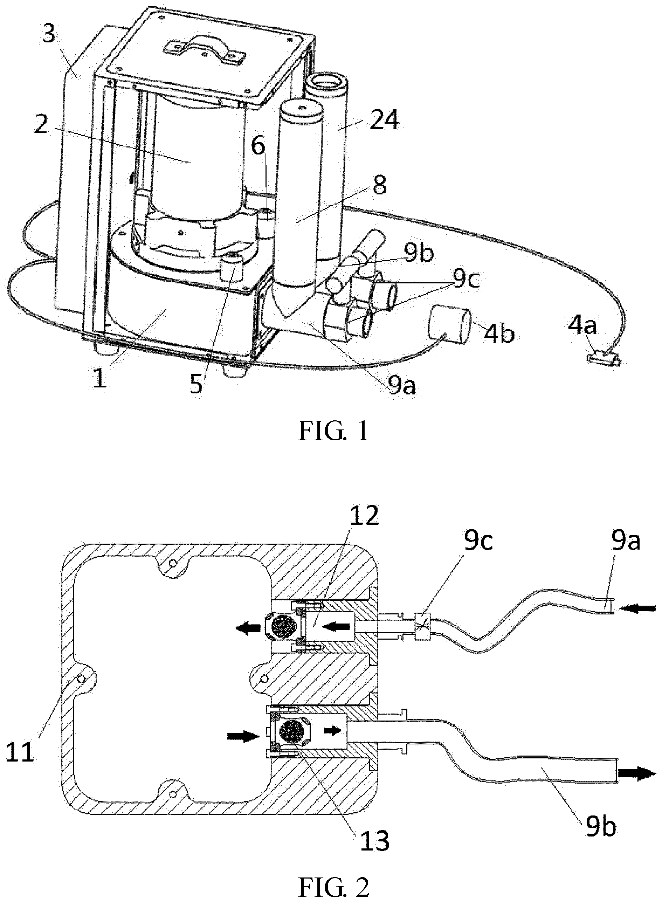

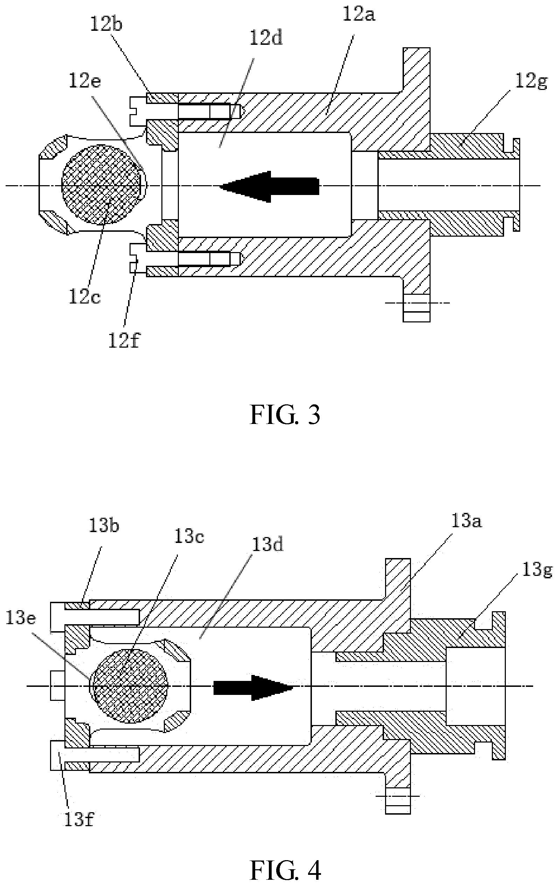

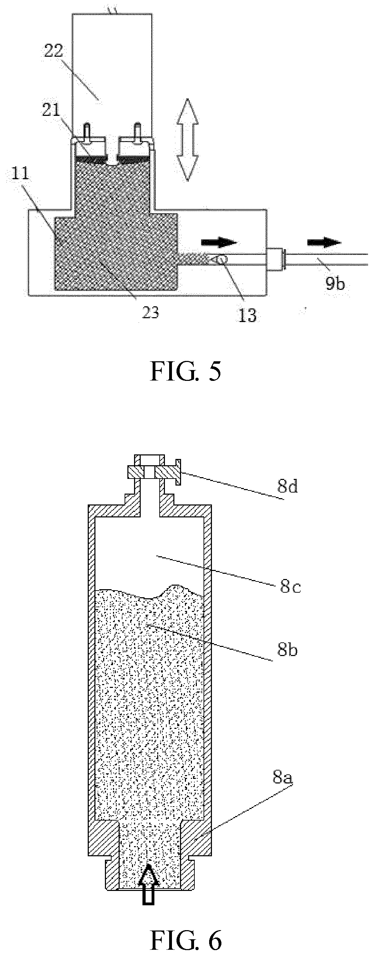

FIG. 1 is a schematic diagram of a fluid driving device according to an embodiment. FIG. 2 is a schematic diagram of a pipe flow system of FIG. 1. FIG. 3 is a schematic diagram of an inflow valve of FIG. 2. FIG. 4 is a schematic diagram of an outflow valve of FIG. 2. FIG. 5 is a schematic diagram of a power system of FIG. 1. Optionally, the fluid driving device is a portable fluid driving device that may provide a periodic pulsating fluid.

As illustrated in FIG. 1, the fluid driving device of the present embodiment includes a base, a pipe flow system 1, a power system 2, and a control system 3. The pipe flow system 1 is configured for providing a fluid channel. The pipe flow system 1 is formed inside the base. The power system 2 is supported on the base and is configured to provide power for the fluid 23 to flow into and out of the pipe flow system 1. And the control system 3 is supported on the base and above the power system 2 and is mainly configured to control and monitor the operation of the fluid driving device. The interior of the entire device is filled with the above-mentioned fluid through a channel of a water injection port 24 before operating the entire device.

For example, a top portion of the control system 3 may be formed as a top plate of the device, and a handle may be arranged above the top plate for the user to conveniently move the fluid driving device. The power system 2 is arranged between the pipe flow system 1 and the control system 3, and the volume of the device may be further reduced by compressing a height between the pipe flow system 1 and the control system 3. In an embodiment, the power system 2 may also be provided on a side of the pipe flow system 1 or may be separately provided, and the control system may also be provided on a side of the pipe flow system 1 or may be separately provided.

As illustrated in FIG. 2, the pipe flow system 1 includes an inner chamber 11 defined by the inner space of the base, an inflow valve 12 for controlling the fluid 23 to flow into the inner chamber, and an outflow valve 13 for controlling the fluid to flow out of the inner chamber. The inflow valve 12 and the outflow valve 13 may both be check valves in order that the fluid 23 flows in only one direction. In some embodiments, the inner chamber of the pipe flow system may be formed by hollowing out the base from the inside, or pipes may be added into the base to form the inner chamber.

As illustrated in FIG. 3, the inflow valve 12 includes a valve body 12a, a ball cage 12b arranged at the valve body 12a, and a ball 12c movable in the ball cage 12b. The valve body 12a is provided with a fluid channel 12d for the fluid 23 to pass through, so as to block or open the fluid channel 12d by the match between the ball 12c and the ball cage 12b. That is, the ball 12c moves inside the ball cage 12b. A direction indicated by an arrow in the figure is the flowing direction of the fluid. The ball cage 12b is arranged at the valve body 12a along the fluid 23 flowing direction and is used to restrict the movement of the ball 12c. The ball cage 12b has an opening 12e of the fluid channel 12d, and a diameter of the ball 12c is larger than that of the opening 12e. As the fluid 23 flows in, the ball 12c is pushed away from the opening 12e of the fluid channel 12d, thereby opening the fluid channel 12d, and in a reverse direction, the ball 12c is pressed against the opening 12e, thereby blocking the fluid channel 12d. Therefore, the fluid 23 can only flow into the valve 12 and cannot flow out in the reverse direction. The ball cage 12b may be fixed to the valve body 12a by screws 12f. The valve body 12a and the fluid channel 12d may be formed of a soft material, for example a polymer material, such as silica gel or rubber.

As illustrated in FIG. 4, the inflow valve 13 includes a valve body 13a, a ball cage 13b arranged at the valve body 13a, and a ball 13c movable in the ball cage 13b. The valve body 13a is provided with a fluid channel 13d for the fluid 23 to pass through, so as to block or open the fluid channel 13d by the match between the ball 13c and the ball cage 13b, that is, the ball 13c moves inside the ball cage 13b. A direction indicated by an arrow in the figure is the fluid 23 flowing direction. The ball cage 13b is arranged at the valve body 13a along the fluid 23 flowing direction and is configured to restrict the movement of the ball 13c. The ball cage 13b has an opening 13e of the fluid channel 13d, and a diameter of the ball 13c is larger than that of the opening 13e. As the fluid 23 flows in, the ball is pushed away from the opening 13e of the fluid channel 13d, thereby opening the fluid channel 13d, and in a reverse direction, the ball is pressed against the opening 13e, thereby blocking the fluid channel 13d. Therefore, the fluid 23 can only flow out of the valve 13 and cannot flow in in the reverse direction. The ball cage 13b may be fixed to the valve body 13a by screws 13f. The valve body 13a and the fluid channel 13d may be formed by the soft material, for example the polymer material, such as the silica gel or rubber.

As illustrated in FIG. 2, the pipe flow system 1 may also be connected to a pipe outside the base, for example, connected to an inflow pipe 9a and an outflow pipe 9b arranged outside the base, where the inflow valve 12 is connected to the inflow pipe 9a (as illustrated in FIG. 3, the inflow valve 12 may be connected to the inflow pipe 9a via a valve quick insertion interface 12g). The outflow valve 13 may be connected to the outflow pipe 9b (as illustrated in FIG. 4, the outflow valve 13 may be connected to the outflow pipe 9b via a valve quick insertion interface 13g). The fluid 23 flows into the inner chamber 11 via the inflow pipe 9a and the inflow valve 12, and flows out of the inner chamber 11 through the outflow valve 13 and the outflow pipe 9b. A flow control valve 9c may also be provided on the inflow pipe 9a for controlling a relationship between the rate of the flow and the pressure of the fluid 23 flowing into the device.

FIG. 5 is a schematic diagram of a power system 2 of FIG. 1. As illustrated in FIG. 5, the power system 2 includes a housing communicated with the upper portion of the inner chamber 11, a piston 21 movable in an upper and down direction inside the housing, and a drive mechanism 22 that is above the housing and is used for driving the piston to move in the upper and down direction.

For example, the housing may have a cylindrical shape, a bottom surface of the piston 21 moving inside the housing is a sealed circular plate, a projection area of the bottom surface of the piston 21 in the up and down direction is S, the drive mechanism 22 may be a linear motor connected to the piston 21 to drive the piston 21 to move up and down. By controlling the downward displacement amount ΔL of the bottom circular plate of the piston 21, the volume change (S × ΔL) of the fluid 23 inside the inner chamber 11 can be controlled, thereby controlling the amount (i.e., cardiac output) of the fluid 23 flowing out of the device, and by controlling a rate of up and down motion driven by the linear motor, a rate (i.e., heart rate) at which fluid 23 flows in and out can be controlled. By such design, the power system 2 can push the fluid 23 to flow out of and into (i.e., to flow into and out of the device) the piping system 1, thereby simulating the contraction and relaxation of the heart.

In an embodiment, the linear motor may be replaced with other drive mechanisms such as cylinders, and other motors, such as electromagnetic motors, solenoid valves, and centrifugal pumps, or other reciprocating mechanical structures. The housing may be in a shape of a cylinder or a rectangular parallelepiped etc.

In an embodiment, the piston 21 and the drive mechanism 22 may be replaced with a deformable elastomer, such as a balloon, and volumetric changes of the fluid 23 inside the inner chamber 11 can be controlled by means of the elastomer deforming in the housing, thereby providing power.

As illustrated in FIG 1, an energy accumulator 8 is further provided between the pipe flow system 1 and the control system 3, and is in communication with the fluid 23 in the inner chamber of the pipe flow system 1 to control a flow damping of the fluid and to adjust a pressure difference between a maximum pressure and a minimum pressure of the fluid.

The structure of the energy accumulator 8 is as illustrated in FIG. 6, and the energy accumulator 8 is of a cavity structure and includes an energy accumulator housing 8a configured for forming a cavity and a valve 8d arranged at a top portion of the housing 8a. The cavity contains the liquid 8b and the air 8c, the volume ratio between the liquid (for example, water) and the air may be adjusted by manually adjusting the valve 8d to perform adjustment, and the energy accumulator is used to adjust a difference value between the first pressure and the second pressure of the fluid, and to eliminate fluid irregular fluctuations to buffer the flow of the fluid. In the present embodiment, the first pressure is a fluid pressure after the energy accumulator stores energy, and the second pressure is a fluid pressure after the energy accumulator releases the energy.

The control system 3 is configured to control the operation of a fluid driving device. For example, the control system 3 may be electrically connected to the power system 2, for example, to provide a control signal (e.g., a voltage or current, etc.) to a drive mechanism 22 (e.g., a linear motor) of the power system 2 such that the power system 2 provides power for the fluid 23 to flow into and out of the piping system 1. For example, the control system may control the movement rate and the displacement change amount of the linear motor to simulate different heart rates and cardiac output.

As illustrated in FIG.1, the fluid driving device further includes a pressure sensor 4, configured to detect a pressure of the fluid 23 in the pipe flow system. For example, the pressure sensor 4 may be arranged at least at one of the outflow pipe and the inner chamber of the pipe flow system 1 to detect the pressure in the inner chamber and the outflow pipe in real time, or may be arranged wherever needed. The control system 3 is configured to control the pressure of the fluid 23 in the pipe flow system 1 based on the pressure of the fluid 23 detected by the pressure sensor 4. For example, control system 3 may control the fluid pressure by controlling the power system 2, or may control the fluid pressure by controlling the inflow valve 12 and the outflow valve 13.

The arrangement of the control system of the device combined with the pressure sensors, temperature sensors and flow sensors allows the device to better control the pressure, temperature, rate of the flow, and the like of the provided pulsating fluid, and to better simulate the heart rate, cardiac output, and the pressure and temperature of the output fluid when used as a simulated heart, so as to better achieve cardiac simulation.

The fluid driving device further includes a heating assembly 5, which is connected to the pipe flow system 1 and is configured to heat the fluid in the pipe flow system. For example, the heating assembly 5 may be a heating rod, is connected to the inner chamber, and is electrically connected to the control system 3, which may provide a control signal to the heating assembly 5 to heat the assembly.

The fluid driving device further includes a temperature sensor 6 configured to detect a temperature of the fluid 23 in the pipe flow system 1. For example, the temperature sensor 6 may be arranged at the inner chamber of the pipe flow system 1 to detect the temperature in the inner chamber in real time, or may be arranged wherever needed. The control system 3 may control the heating assembly 5 based on the detection result of the temperature sensor, for example, when the temperature sensor detects a low temperature, the control system 3 may control the heating rod to heat the fluid 23 in the inner chamber to maintain the fluid within a set range of temperature.

The fluid driving device may further include a flow sensor configured to detect the rate of the flow of the fluid in the pipe flow system. The flow sensor may be arranged on the inflow pipe and the outflow pipe, or at the inflow valve 12 and the outflow valve 13 to detect the rate of the flow of the fluid flowing into the pipe flow system in real time. The control system 3 is configured to control the rate of the flow of the fluid flowing into the pipe flow system 1 based on the rate of the flow of the fluid detected by the flow sensor. For example, the control system 3 may control the rate of the flow of the incoming fluid by controlling the power system 2, or may control the rate of the flow of the incoming fluid by controlling the inflow valve 12, and may also control the rate of the flow of the incoming fluid by controlling the flow valve.

In an embodiment, the control system 3 is further configured to stop the power system 2 and the heating assembly 5 from operating when detecting no fluids in the pipe flow system 1, thereby achieving self-diagnosis and protection of the simulated cardiac devices. In an embodiment, the fluid driving device may further include an alarm that can send an alert to the user when detecting no fluids in the pipe flow system 1.

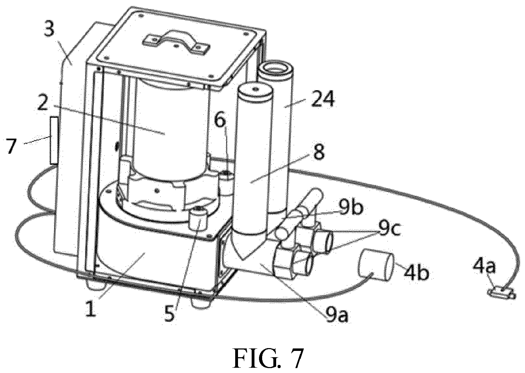

In an embodiment, the fluid driving device further includes a touch display screen 7, and as illustrated in FIG. 7, the touch display screen 7 is electrically connected to the control system 3 and may be arranged on a sidewall of the control system 3. In an embodiment, the touch display screen 7 may be wirelessly connected, and may be a portable computer. The touch display screen 7 may display specific parameters of a power source of the power system 2, the temperature and pressure, and the like of the fluid 23 detected by the pressure sensor 4 and the temperature sensor, and may also be used by the user to input a control command to provide a control signal to the power system 2, the heating assembly 5, and the like via the control system. By such design, the pressure and temperature of the fluid 23 can be monitored in real time by using the touch display screen, thereby facilitating the determination as to whether the pressure and temperature need to be adjusted, and to more easily realize control (for example, controlling the amplitude, frequency, and the like of the motor) of the power system, temperature control of the fluid 23, and the like, thereby better performing simulation as needed.

In an embodiment, the control system 3 may implement the different functions described above by a single control device, or may implement the different functions described above by a plurality of control devices. The control system 3 may include a first control device configured to control the pressure of the fluid 23 in the pipe flow system 1 based on the pressure of the fluid 23 detected by the pressure sensor. The control system 3 may include a second control device configured to control the heating assembly 5 based on the detection result detected by the temperature sensor. The control system 3 may include a third control device configured to control the rate of the flow of the fluid 23 flowing into the pipe flow system 1 based on the rate of the flow of the fluid 23 detected by the flow sensor. The control system 3 may include a fourth control device, which is configured to stop the power system 2 and the heating assembly 5 when detecting no fluids in the pipe flow system 1, and may also be electrically connected to the alarm to control the provision of the alarm. These control devices can be integrated on one chip or may be implemented by multiple chips.

The fluid driving device provided by the present embodiment can generate periodic pulsating fluid, can control a periodic rate of the flow of the fluid, the periodic pressure and the temperature according to actual needs, simulates the contraction and relaxation of the heart, and may be used for medical teaching (for example, for simulated surgery), medical device testing (for example, testing prosthetic heart valves), assist medical treatment (blood vascular system provides pulsating pressure and blood with temperature of 37 ± 2 degrees Celsius) and other situations requiring periodic pulsating fluids.

### INDUSTRIAL APPLICABILITY

The fluid driving device of the present disclosure has an overall simple and compact structure, which significantly reduces the overall instrument volume and weight and is convenient to carry around.

## Claims

1. A fluid driving device, comprising:
a pipe flow system, configured to provide a fluid channel;
a power system, configured to provide power for a fluid to flow into and out of the pipe flow system; and
a control system, configured to control operation of the fluid driving device.

2. The fluid driving device of claim 1, further comprising a base, wherein the pipe flow system is formed inside the base, the power system is supported on the base, the control system is supported on the base, and the power system is disposed between the control system and the base.

3. The fluid driving device of claim 2, wherein the pipe flow system comprises an inner chamber defined by an interior space of the base, an inflow valve operative to control the fluid to flow into the inner chamber, and an outflow valve operative to control the fluid to flow out of the inner chamber.

4. The fluid driving device of claim 3, wherein the inflow valve and the outflow valve are both check valves.

5. The fluid driving device of claim 4, wherein the check valve comprises a valve body, a ball cage arranged at the valve body along a flowing direction of the fluid, and a movable ball arranged inside the ball cage, wherein the valve body comprises a fluid channel, and the ball is configured to block or open the fluid channel by moving in the ball cage.

6. The fluid driving device of claim 5, wherein the valve body is formed from a soft material, the soft material comprising silicone or rubber.

7. The fluid driving device of claim 3, further comprising an energy accumulator, which is communicated with the interior chamber of the pipe flow system and is configured to buffer the flow of the fluid and to adjust a pressure difference between a first pressure and a second pressure of the fluid, wherein the first pressure is a fluid pressure after the energy accumulator stores energy, and the second pressure is a fluid pressure after the energy accumulator releases energy.

8. The fluid driving device of claim 3, wherein the power system comprises:
a housing, communicated with an upper portion of the inner chamber;
a piston, movable along an up and down direction in the housing; and
a drive mechanism, disposed above the housing and configured to drive the piston to move in the up and down direction.

9. The fluid driving device of claim 8, wherein the drive mechanism comprises a linear motor, a cylinder, a solenoid valve, or a reciprocating mechanical structure.

10. The fluid driving device of claim 3, wherein the power system comprises:
a housing, communicated with an upper portion of the inner chamber; and
a deformable elastomer, connected to the housing and configured to deform in the housing so as to provide a power.

11. The fluid driving device of claim 3, further comprising a pressure sensor configured to detect a pressure of the fluid in the pipe flow system.

12. The fluid driving device of claim 11, wherein the control system is configured to control the pressure of the fluid in the pipe flow system based on the pressure of the fluid detected by the pressure sensor.

13. The fluid driving device of claim 3, further comprising a flow sensor configured to detect a rate of flow of the fluid flowing into the pipe flow system.

14. The fluid driving device of claim 13, wherein the control system is configured to control the rate of flow of the fluid flowing into the pipe flow system based on the rate of flow of the fluid detected by the flow sensor.

15. The fluid driving device of claim 11, further comprising a heating assembly connected to the pipe flow system and configured to heat the fluid in the pipe flow system.

16. The fluid driving device of claim 15, further comprising a temperature sensor configured to detect a temperature of the fluid in the pipe flow system.

17. The fluid driving device of claim 16, wherein the control system is further configured to control the heating assembly depending on a detection result of the temperature sensor.

18. The fluid driving device of claim 3, wherein the inflow valve is connected to an inflow pipe, the outflow valve is connected to an outflow pipe, wherein the inflow pipe is provided with a flow control valve configured to control the rate of flow and pressure of the fluid flowing into the fluid driving device.

19. The fluid driving device of claim 16, further comprising a touch display screen or a portable computer electrically connected to the control system and configured to input a control command and to display the pressure and the temperature detected by the pressure sensor and the temperature sensor.

20. The fluid driving device of claim 17, wherein the control system is further configured to control the power system and the heating assembly to stop operating in response to detecting no fluids in the pipe flow system.
